(19) Europäisches Patentamt
European Patent Office
Office européen des brevets

(11) Publication number: 0 240 094
A2

# (12) EUROPEAN PATENT APPLICATION

(21) Application number: 87300026.9

(22) Date of filing: 05.01.87

(51) Int. Cl.[3]: C 07 C 125/065
C 07 C 119/042, C 07 C 118/00

(30) Priority: 06.01.86 US 816550

(43) Date of publication of application:
07.10.87 Bulletin 87/41

(84) Designated Contracting States:
BE DE FR GB IT NL

(71) Applicant: THE DOW CHEMICAL COMPANY
2030 Dow Center Abbott Road P.O. Box 1967
Midland, MI 48640(US)

(72) Inventor: Chang, Kuo Yuan
2708 Mt.Vernon
Midland Michigan 48640(US)

(74) Representative: Raynor, John et al,
W.H. Beck, Greener & Co 7 Stone Buildings Lincoln's Inn
London WC2A 3SZ(GB)

## (54) Process for preparing isocyanatoalkyl esters.

(57) A three-step reaction process for ultimately preparing isocyanatoalkyl esters. The first step involves the reaction of an amino alcohol and dimehtyl, diethyl or dipropyl carbonate to produce an N-hydroxy-alkyl-O-alkyl urethane. The second step may involve the reaction of the already produced N-hydroxy-alkyl-O-alkyl urethane with an ester of a carboxylic acid in the presence of a catalyst such as 1,4-diazabicyclo-(2, 2, 2)octane to produce a urethano ester in the form of an alcohol blocked isocyanatoalkyl ester. The third step may involve the pyrolysis of the already produced urethano ester to yield an isocyanatoalkyl ester. Each step of the reaction may be preformed separately or in sequence.

EP 0 240 094 A2

# PROCESS FOR PREPARING ISOCYANATOALKYL ESTERS

The present invention relates to a process for preparing isocyanatoalkyl esters of organic carboxylic acids, particularly β-isocyanatoethyl methacrylate (IEM) and alcohol blocked isocyanatoalkyl esters, particularly methanol blocked IEM.

Isocyanatoalkyl esters are a known class of compounds having utility in polymer production. For example, IEM is known to undergo addition polymerization to produce soluble, thermoplastic resinous polymers which are used as a treatment for wool to render it resistant to shrinkage as taught in U.S. Patent No. 2,718,516 (Bortnick). Isocyanatoalkyl esters are also characterized by a strong tendency to polymerize with one another or with other unsaturated compounds to form useful copolymers as taught in U.S. Patent No. 2,821,544 (Holtschmidt).

The Bortnick and Holtschmidt patents also disclose methods for producing isocyanatoalkyl esters. Bortnick discloses producing isocyanato esters having the general formula Z - COO - A - NCO, where A is an alkylene group, $C_nH_{2n}$ in which n is 2 to 14, and Z is an acrylic, methacrylic, chloroacrylic or crotonic radical. The Bortnick process involves a three-step

reaction in which the first step is the reaction of a chloroformate with an ethanolamine to produce a hydroxycarbonate. The hydroxycarbonate is, then, reacted with an acrylyl chloride in the presence of acrylonitrile to form a solid urethano ester. The third step involves reacting the urethano ester with phosphorous pentachloride to produce the isocyanatoalkyl ester. As an alternative, Bortnick at Col. 6 discloses that the last step may be a pyrolysis one when the carbon atom adjacent to the isocyanate group of the intermediate is secondary or tertiary carbon atom.

Various chloride by-products are produced in Bortnick's three-step process. As such, it is necessary to use hydrogen chloride scavengers to remove those impurities. See, for example, U.S.Patent No. 4,310,688 (Mendoza) which discloses reducing the level of hydrolyzable chloride-containing impurities in an isocyanatoalkyl ester by reacting the impurities with a vicinal epoxide-containing compound and then fractionally distilling the mixture to recover the isocyanatoalkyl ester.

Holtschmidt discloses the formation of isocyanate esters having the general formula $R - COO- (CH_2)_n - NCO$. where R represents a mono-valent aliphatic unsaturated hydrocarbon and n is from 1-4, by esterifying unsaturated carboxylic acids such as acrylic, methacrylic acid or crotonic acid with chlorohydrates of amino alcohols. The resulting hydrochloride intermediates are then reacted with phosgene to produce the isocyanatoalkyl ester end product. Not only does this process produce chloride--containing impurities as in Bortnick, but it also uses poisonous phosgene gas.

Phosgene is also used in the process of Burdett, assigned to Dow Chemical Company, the same assignee as the present invention. Thus, Burdett discloses preparation of isocyanatoalkyl esters of organic carboxylic acids by reacting an aqueous solution of a 2-oxazoline with an organic solution of phosgene dissolved in a water-immiscible organic solvent in the presence of a hydrochloric acid acceptor. Preliminary steps for producing the 2-oxazoline starting material may be involved. Dow's current process for producing IEM may, therefore be set forth as follows:

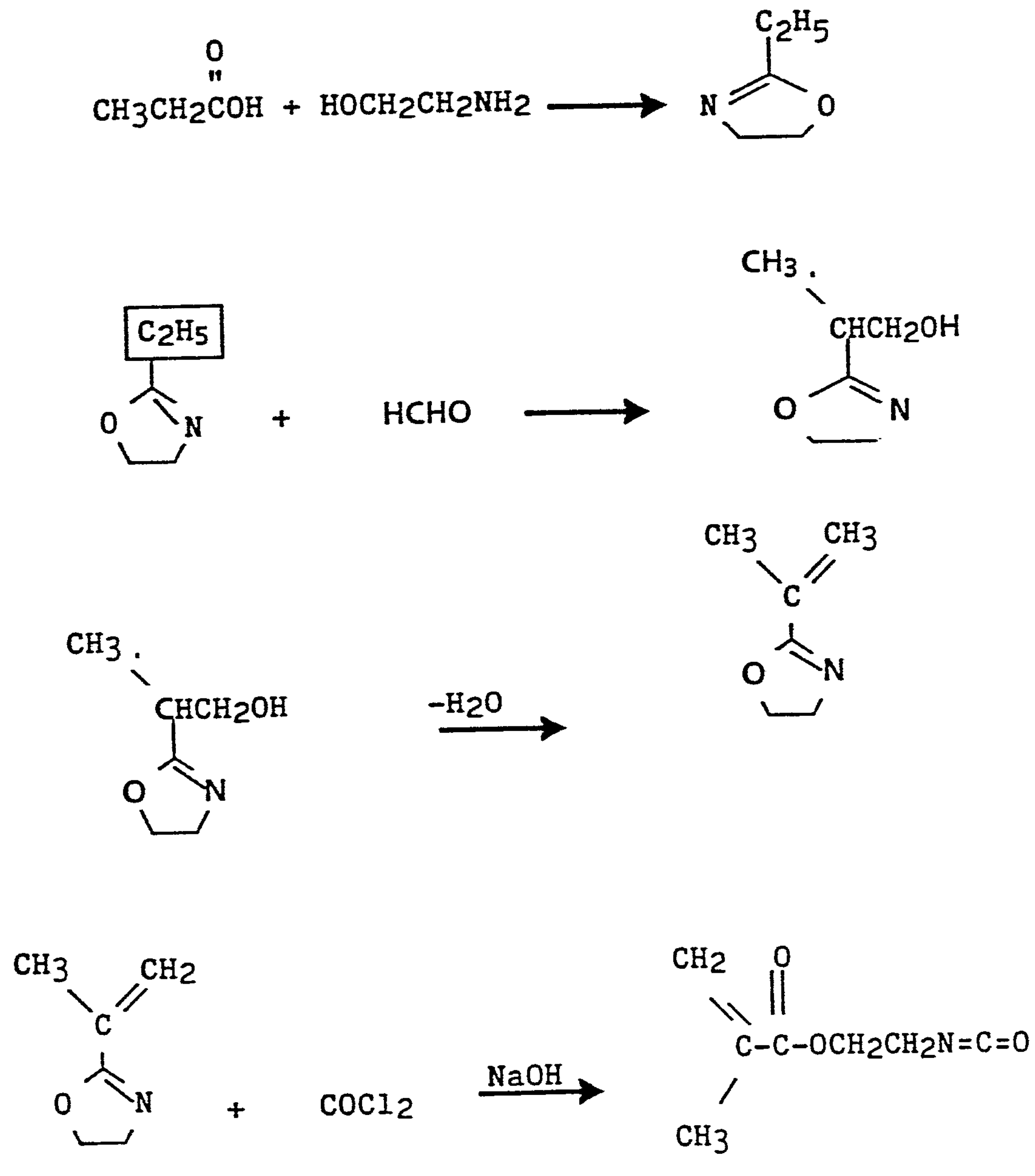
-H2O
NaOH

As indicated with regard to the Bortnick and Holtschmidt methods, chlorine-containing impurities are present in Dow's current process, but may be removed by the process of U.S. Patent No. 4,310,688 (Mendoza). Other impurities may also be introduced by the reaction sequence set forth above. Those impurities may also be removed. See U.S. Patent No. 4,260,811 (Mendoza) directed to reducing the level of 2-oxazoline or oxazine impurities by reacting the impurity with an organic acid chloride, a chloroformate, a thiochloroformate, a sulfonyl chloride, phosphoryl chloride, sulfuryl chloride, phosphoryl chloride, sulfuryl chloride or thionyl chloride and fractionally distilling the reaction mixture to recover purified isocyanatoalkyl ester.

As can be seen, the prior art processes for producing isocyanatoalkyl esters all have one or more drawbacks. Accordingly, there exists the need for a process for preparing isocyanatoalkyl esters of organic carboxylic acids which avoids the use of phosgene and other toxic chemicals and which produces very low organic wastes or impurities. Likewise, it would be desirable to have a process which uses readily available and relatively inexpensive starting materials.

In accordance with the present invention, there is provided isocyanatoalkyl esters and blocked isocyanatoalkyl esters by a process which uses readily available starting materials, avoids the use of toxic reactants, and produces low organic wastes. The isocyanatoalkyl esters prepared by the present process are represented by the formula

$$R_2 - \overset{\overset{\displaystyle O}{\|}}{C} - O - R_1 - N{=}C{=}O \qquad (I)$$

where $R_1$ is a straight or branched chain alkylene group having from two to four carbon atoms and $R_2$ is a straight of branched chain alkyl group having from one to four carbon atoms.

The instant process of preparing isocyanatoalkyl esters of this type involves a three-step reaction mechanism. In the first step an amino alcohol having the formula

$$HO - R_1 - NH_2 \qquad (II)$$

where $R_1$ is as in formula (I), is reacted preferably with dimethyl carbonate. Alternatively, diethyl or dipropyl carbonate may be used although certain problems may be encountered when doing so. No catalyst or solvent is required. The resulting product is a lower alkyl urethane, preferably a liquid methyl urethane, more specifically a N-hydroxalkyl-O-alkyl urethane and preferably N-hydroxyalkyl-O-methylmethane, having the formula

$$HO - R_1 - HN - \overset{\overset{\displaystyle O}{\|}}{C} - R_3 \qquad (III)$$

where R1 is as in formula (I) and R3 is methyl, ethyl or propyl. The urethane of formula (III) can be used directly in the next step without any purification; although, it may be isolated and removed if desired.

In the second step, then, the urethane of formula (III) is reacted with an ester of a carboxylic acid, such as a readily available methacrylate or acrylate, to produce an urethano ester having the formula

$$R2 - \overset{O}{\overset{\|}{C}} - O - R1 - NH - \overset{O}{\overset{\|}{C}} - R3 \qquad (IV)$$

where R1 and R2 are as in formula (I), and R3 is as in formula (III). In this step a catalyst such as 1,4-diazabicyclo(2,2,2)octane (DABCO) is preferred since it results in much higher yields than other catalysts. However, other tertiary amines having a suitable boiling point (i.e. higher than the reaction temperature) may also be used. No toxic hydrogen chloride scavengers are required.

The reaction sequence can be stopped at this point since the urethano ester of formula (IV) has utility apart from being a precursor to the isocyanatoalkyl ester. That is, from one perspective. the urethano ester of formula (IV) may be viewed as an alcohol blocked isocyanatoalkyl ester which can itself be used in polyurethane coating applications and can be converted to other blocked derivatives. It is readily separated by vacuum distillation for subsequent use. It can also be deblocked by alcohol removal to produce the isocyanatoalkyl ester of formula (I). That is the third step in the process.

The third step involves pyrolyzing the urethano ester of formula (IV) in the presence of a silicate such as sodium silicate, magnesium silicate, etc. The

silicate appears to act as a catalyst for methanol removal. An inorganic base such as disclosed in Bortnick, U.S. Patent No. 2,718,516 (see Col. 3, lines 15-19) may also be used, but is not preferred. The pyrolysis is preferably performed in an inert gas stream, such as hydrogen, nitrogen, argon, helium, etc., to prevent recombination of alcohol and the isocyanatoalkyl ester. The third step is also temperature dependent. At temperatures from 175 to 400°C there occurs a pyrolysis which is highly selective (the higher end of the range being more so than the lower end) toward the production of the isocyanatoalkyl ester of formula (I).

As such, the overall process can be expressed as follows:

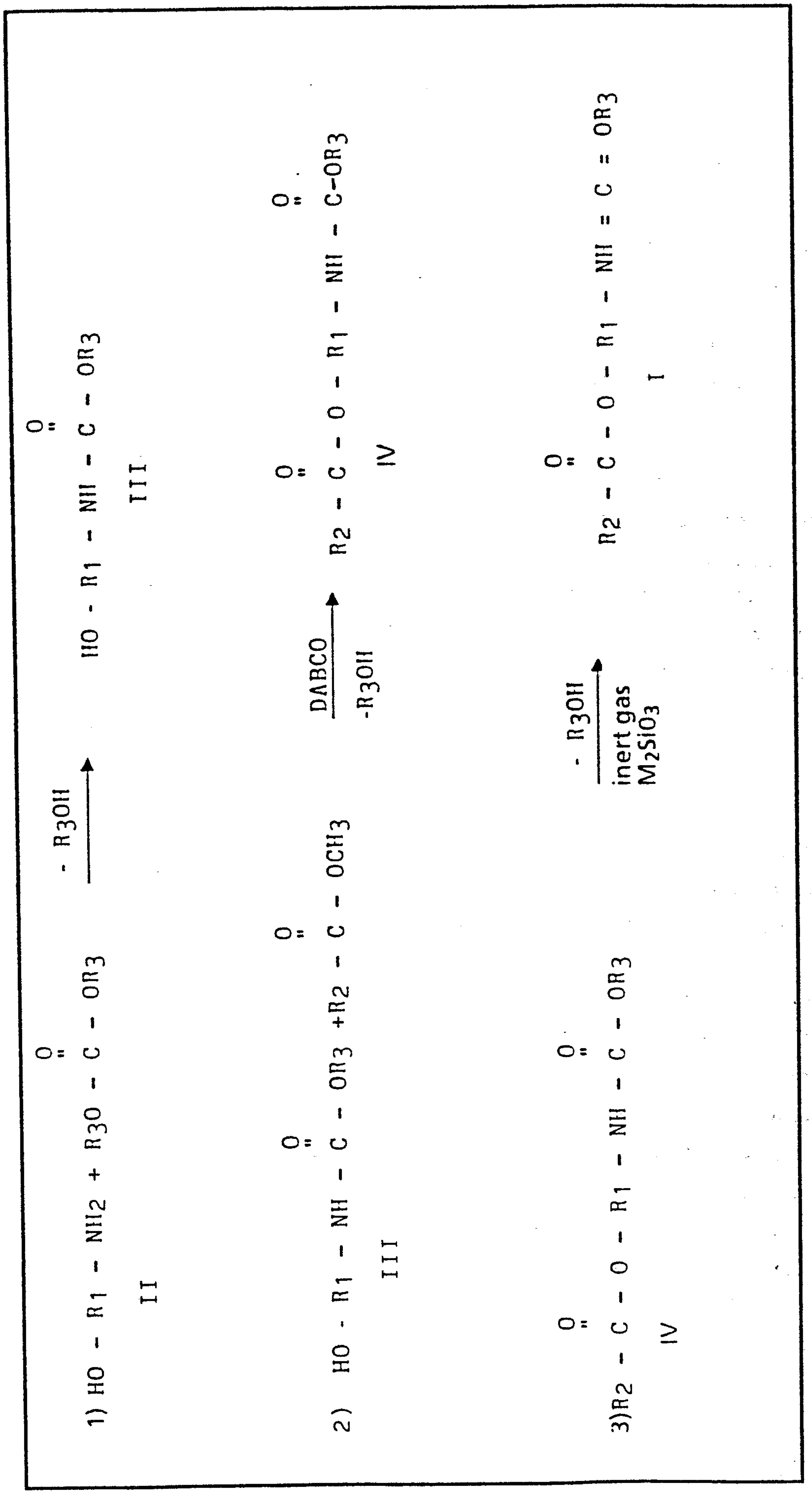
1)
II
- $R_3OH$
III
2)
III
DABCO
-$R_3OH$
IV
3)
IV
- $R_3OH$
inert gas
$M_2SiO_3$
I

As a result, high yields of isocyanatoalkyl esters and blocked isocyanatoalkyl esters are obtained. Fewer steps are required than are found in the presently commercial processes; the reaction is salt free; toxic reactants are avoided, and there are no chloride-containing impurities to be removed.

Accordingly, the present invention to provides an improved process for preparing isocyanatoalkyl esters and the intermediates therefor.

The preferred process can actually be viewed as three separate processes which can be performed individually or which can be combined as a three-step reaction process to produce isocyanatoalkyl esters. Therefore, the preferred embodiment, and any alternative embodiments, for each step of the process will be dealt with individually.

The first step is preferably carried out at room temperature to 100°C. Above 100°C the yield of N-hydroxyalkyl-O-methyl urethane is decreased and production of 2-oxazolidinone increases. The amino alcohol of formula (II), which is preferably monoethanolamine, is allowed to react with excess dimethyl carbonate (the preferred carbonate) at the mild temperatures mentioned. The excess dimethyl carbonate is used to compensate for its loss during methanol removal because of azeotrop formation. No catalyst or solvent is required and the reaction should be allowed to proceed for one to three days. The methanol and excess dimethyl carbonate may be removed under vacuum to give the urethane of formula (III), namely N-hydroxyalkyl-O-methyl urethane. The yield is around 90 percent. That urethane may be purified and

used in the remaining process steps or may be used as a precursor in other reaction mechanisms. If it is to be used in the second step of the instant process, it is not necessary that it be separated and purified. Rather, it may be used directly in the next step.

The second step may utilize the urethane of formula (III) as prepared in step one, or a similar urethane prepared by other processes such as that taught by Bortnick, U.S. Patent No. 2,718,516. In Bortnick (see Example I), an amino alcohol is reacted with potassium carbonate and a chloroformate. Bortnick produces an N-hydroxyalkyl-O-ethyl urethane can be produced by a similar process. Of course, in that instance it would be necessary to remove the chloride-containing impurities prior to proceeding.

In any event, the urethane of formula (III) may, as a second step, be reacted with an ester of a carboxylic acid, preferably methyl methacrylate or methyl acrylate, to produce the urethano ester of formula (IV). Methyl methacrylate and methyl acrylate are readily available. The carboxylic acid ester is added to the urethane of formula (III) along with a catalyst which is preferably 1,4-diazabicyclo(2,2,2)-octane (DABCO). With (DABCO) yields of around 80 percent are possible; whereas, with a sodium methoxide catalyst the yields drops off to only around 26 percent. The mixture is stirred and heated to distill the methanol formed during the transesterification reaction. Preferred temperatures are 60 to 110°C for a sufficient time to completely remove the methanol. The resulting urethano ester of formula (IV),which may be purified by vacuum distillation for subsequent uses. As mentioned, the urethano ester of formula (IV) can be

considered an alcohol (preferably a methanol) blocked isocyanatoalkyl ester. A methanol blocked isocyanatoalkyl ester is an easily handleable liquid and may be used in polyurethane production as well as in step three of the present process.

In step 3, the urethano ester of formula (IV) as produced in step 2 may have a methanol block removed by pyrolysis. Alternatively, a urethano ester prepared by other processes may be used in step 3. For example, Bortnick, U.S. Patent No. 2,718,516 teaches preparing urethano esters by reacting a hydroxycarbonate with acrylyl chloride in the presence of an acrylonitrile. While the urethano ester formed by Bortnick is a solid ethanol blocked one, presumably a methanol blocked one similar to formula (IV) can be prepared by a similar process. Bortnick also suggests pyrolysis as a possibility for producing an isocyanatoalkyl ester from his urethano ester (see col. 6). However, he suggests doing so in the presence of an inorganic basic compound and only when the carbon atom adjacent to the isocyanate group is a secondary or tertiary carbon atoms.

In the present process, the pyrolysis is preferably conducted in the presence of a silicate and a primary carbon atom is preferably found adjacent the isocyanate group. Even though this is the case, it has been found that it is possible at controlled temperatures, using hydrogen gas stream, to convert the urethano ester of formula (IV) to the isocyanatoalkyl ester of formula (I). The yields are temperature dependent. As mentioned the preferred range is 175 to 400°C. Yields of 80 to 90 percent can be obtained. The

isocyanatoalkyl ester may be isolated by vacuum distillation.

The following example is illustrative.

## Example

1) To 130g dimethyl carbonate was added dropwise 61g monoethanolamine. The mixture was allowed to stand at room temperature for three days. The methanol and unreacted dimethyl carbonate were removed at 45°C using a rotary evaporator under reduced pressure to give 115.2g crude N-hydroxyethyl-O-methyl urethane (about at 90 percent yield).

2) six grams of the crude urethane from step 1) was mixed with 17.9 g methyl methacrylate and 0.2 g DABCO. The mixture was stirred and heated to distill the methanol formed during the transesterification reaction. The distillation was stopped when the pot temperature reached 105°C in 1.5 hours. The distillation yileds was 3.14 g distillate. The mixture left in the pot weighted 20.45 g. The mixture was analyzed by gas chromatograph using an internal standard and showed a 79.88 percent yield of methanol blocked β-isocyanatoethyl methacrylate (MBIEM) (i.e. urethano ester. It was purified by vacuum distillation.

3) A dilute solution of the MBIEM, produced by step 2, in cyclohexane, was injected into the injector-splitter of a 30-meter SE-54 capillary gas chromatograph column. The splitter had been treated with a dilute sodium silicate solution. A gas flow of 1 ml/min of hydrogen was maintained as the temperature was increased. At 300°C 10 percent β-isocyanatoethyl methacrylate (IEM) was produced while approximately 90 percent of the composition remained MBIEM. At 400°C

over 50 percent of IEM was produced, while the MBIEM level fell to about 40 percent.

While the methods herein described constitute preferred embodiments of the invention, it is to be understood that the invention is not limited to these precise methods and that changes may be made in the method without departing from the scope of the invention, which is defined in the appended claims.

## CLAIMS

1. A process for preparing a urethano ester having the formula

$$R_2 - \overset{\displaystyle O}{\overset{\|}{C}} - O - R_1 - NH - \overset{\displaystyle O}{\overset{\|}{C}} - OR_3 \qquad (IV)$$

where $R_1$ is a straight or branched chain alkylene group having from two to four carbon atoms, $R_2$ is a straight or branched chain alkyl group having from one to four carbon atoms, and $R_3$ is methyl, ethyl, or propyl, comprising the steps of:

1) reacting an amino alcohol having the formula $HO-R_1-NH_2$ (II) where $R_1$ is as previously defined with a dimethyl, diethyl, or dipropyl carbonate to produce a urethane having the formula:

$$HO - R_1 - HN - \overset{\displaystyle O}{\overset{\|}{C}} - OR_3 \qquad (III)$$

where $R_1$ is a straight or branched chain alkylene group having two to four carbon atoms and $R_3$ is as defined above, and

2) reacting said urethane with an ester of a carboxylic acid to form the urethano ester having the formula (IV) as defined above.

2. A process as claimed in Claim 1 wherein in step

1) said carbonate is dimethyl carbonate and amino alcohol is monoethanolamine.

3. A process as claimed in Claim 1 or Claim 2, wherein said ester of a carboxylic acid in step 2) is methylmethacrylate.

4. A process as claimed in any one of the preceding claims wherein step 2) is conducted in the presence of a 1,4-diazabicyclo(2,2,2)-octane catalyst.

5. A process as claimed in any one of the preceding claims wherein step 1) is carried out at a temperature from room temperature to 100°C.

6. A process as claimed in any one of the preceding claims wherein in step 2) the reaction mixture is heated at a temperature from 60°C. to 110°C to distill methanol formed during the transesterification reaction.

7. A process for preparing an isocyanatoalkyl ester organic carboxylic acid having the formula

$$R_2 - \overset{O}{\overset{\|}{C}} - O - R_1 - N{=}C{=}O \qquad (I)$$

where $R_1$ and $R_2$ are as defined in Claim 1 which process comprises pyrolysing a urethano ester of the formula

$$R2 - \overset{O}{\overset{\|}{C}} - O - R_1 - NH - \overset{O}{\overset{\|}{C}} - OR_3 \qquad (IV)$$

where $R_1$ is a straight or branched chain alkylene group having from two to four carbon atoms, $R_2$ is a straight or branched chain alkyl group having from one to four carbon atoms, and $R_3$ is methyl, ethyl, or propyl, in the presence of a silicate and an inert gas stream to form an isocyanatoalkyl ester having formula (I).

8. A process as claimed in Claim 7, wherein the silicate is sodium silicate and the isocyanatoalkyl ester is β-isocyanatoethyl methacrylate.

9. A process as claimed in Claim 7 or Claim 8, wherein the pyrolysis reaction is carried out at a temperature from 175°C to 400°C.

10. A process as claimed in any one of Claims 7 to 9, wherein the compound of the formula (IV) has been prepared by a method as claimed in any one of Claims 1 to 6.

11. A process for preparing an isocyanatoalkyl ester organic carboxylic acid having the formula

$$R_2 - \overset{\overset{\displaystyle O}{\|}}{C} - O - R_1 - N{=}C{=}O \qquad (I)$$

where $R_1$ is a straight or branched chain alkylene group having from two to four carbon atoms and $R_2$ is a straight or branched chain alkyl group having from one to four carbon atoms comprising the steps of:

1) reacting an amino alcohol having the formula $HO\text{-}R_1\text{-}NHR_2$ (II) where $R_1$ is as previously defined with a dimethyl, diethyl, or dipropyl

carbonate to produce a urethane having the formula:

$$HO - R_1 - HN - \overset{\overset{O}{\|}}{C} - OR_3 \qquad (III)$$

where $R_1$ is a straight or branched chain alkylene group having two to four carbon atoms, and $R_3$ is methyl, ethyl, or propyl,

2) reacting said urethane with an ester of a carboxylic acid to form a urethano ester having the formula

$$R2 - \overset{\overset{O}{\|}}{C} - O - R_1 - NH - \overset{\overset{O}{\|}}{C} - OR_3 \qquad (IV)$$

where $R_1$ and $R_3$ are as previously defined, and $R_2$ is a straight or branched chain alkyl group having from one to four carbon atoms, and

3) pyrolyzing said urethano ester in the presence of a silicate and an inert gas stream to form an isocyanatoalkyl ester having formula (I).